(19) Europäisches Patentamt
European Patent Office
Office européen des brevets

(11) EP 1 347 059 A1

(12) **EUROPEAN PATENT APPLICATION**

(43) Date of publication:
**24.09.2003 Bulletin 2003/39**

(51) Int Cl.[7]: **C12Q 1/37**, G01N 33/68, A61K 31/00, A61K 38/00

(21) Application number: **02006264.2**

(22) Date of filing: **20.03.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Biofrontera Pharmaceuticals AG**
**51377 Leverkusen (DE)**

(72) Inventors:
- **Lübbert, Hermann, Prof.Dr.**
  **51381 Leverkusen (DE)**
- **Engels, Peter, Dr.**
  **51429 Bergisch Gladbach (DE)**

(74) Representative:
**Sternagel, Fleischer, Godemeyer & Partner**
**Patentanwälte**
**Braunsberger Feld 29**
**51429 Bergisch Gladbach (DE)**

(54) **Cathepsin Y for the development of a medicament for the treatment of stroke**

(57) The present invention relates to the use of a polynucleotide sequence encoding Cathepsin Y or a amino acid sequence of Cathepsin Y protein for the characterisation or identification of therapeutic agents for stroke, the use of such sequences for the development of a medicament, and agents useful as medicaments for the treatment of stroke.

EP 1 347 059 A1

## Description

The present invention relates to the use of a polynucleotide sequence encoding Cathepsin Y or an amino acid sequence of Cathepsin Y protein for the characterisation or identification of therapeutic agents for stroke, the use of such sequences for the development of a medicament, and agents useful as medicaments for the treatment of stroke.
Stroke is a rather common and potentially harmful event that often leaves patients severely disabled for the rest of their lives. It is the result of either an interruption of blood and therefore oxygen supply to the brain, or bleeding in the brain that occurs when a blood vessel bursts. The reduced oxygen supply leads to shortage of energy in the affected brain regions and results in the death of neurons around the lesion. The degeneration spreads from the site that is affected by the reduced blood supply into regions which have at all times obtained sufficient oxygen.
Stroke results from a loss of blood flow to the brain caused by thrombosis or haemorrhage. With an incidence of 250-400 in 100.000 and a mortality rate of around 30%, stroke is a major public health problem. About one-half of the stroke survivors suffer from significant persisting neurological impairment and/or physical disability. Thus, the economic costs of stroke amount to many billions of dollars worldwide.
A stroke occurs when the blood supply to part of the brain is suddenly interrupted or when a blood vessel in the brain bursts. As a consequence, brain cells die when they no longer receive oxygen and nutrients from the blood or when they are damaged by sudden bleeding into the brain. Some brain cells die immediately after interruption of the blood flow into the brain, while others remain at risk for death and stay in a compromised state for hours. These damaged cells make up the so-called "ischemic penumbra", and with timely treatment these cells could be saved. Stroke ultimately leads to infarction, the death of huge numbers of brain cells, which are eventually replaced by a fluid-filled cavity (or infarct) in the injured brain.
There are two major forms of stroke: ischemic - blockage of a blood vessel supplying the brain, and hemorrhagic - bleeding into or around the brain. An ischemic stroke can be caused by a blood clot (embolus or thrombus), which is blocking a vessel. It can also be caused by the narrowing of an artery due to the build-up of plaque (a mixture of fatty substances, including cholesterol and other lipids). The underlying pathological process called stenosis is often observed in arteriosclerosis, the most common blood vessel disease. About 80% of all strokes are ischemic strokes. A hemorrhagic stroke is caused by the bursting of an artery in the brain. Subsequently, blood spews out into the surrounding tissue and upsets not only the blood supply but also the delicate chemical balance neurons require to function. Hemorrhagic strokes account for approximately 20% of all strokes.
A transient ischemic attack (TIA), sometimes called a mini-stroke, starts just like a stroke but then resolves leaving no noticeable symptoms or deficits. The occurrence of a TIA is a warning that the person is at risk for a more serious and debilitating stroke. About one-third of patients who have a TIA will have an acute stroke sometime in the future. The addition of other risk factors compounds a person's risk for a recurrent stroke. The average duration of a TIA is a few minutes. For almost all TIAs, the symptoms go away within an hour. There is no possibility to distinguish whether symptoms will be just a TIA or persist and lead to death or disability.
Recurrent stroke is frequent; about 25 percent of people who recover from their first stroke will have another stroke within 5 years. Recurrent stroke is a major contributor to stroke disability and death, with the risk of severe disability or death from stroke increasing with each stroke recurrence. The risk of a recurrent stroke is greatest right after a stroke, with the risk decreasing with time. About 3 percent of stroke patients will have another stroke within 30 days of their first stroke and one-third of recurrent strokes take place within 2 years of the first stroke.
The most important risk factors for stroke are hypertension, arteriosclerosis, heart disease, diabetes, and cigarette smoking. Others include heavy alcohol consumption, high blood cholesterol levels, illicit drug use, and genetic or congenital conditions, particularly vascular abnormalities. People with multiple risk factors compound the destructive effects of these risk factors and create an overall risk greater than the simple cumulative effect of the individual risk factors.
Although stroke is a disease of the brain, it can affect the entire body. Depending on the affected brain region and the severity of the attack, post-stroke patients suffer from a variety of different symptoms. Some of the disabilities that can result from stroke include paralysis, cognitive deficits, speech problems, emotional difficulties, daily living problems, and pain. Stroke disability is devastating to the stroke patient and family. but therapies are available to help rehabilitate post-stroke patients. The mortality rate observed in ischemic stroke is around 30%. The time window for a medical treatment is narrow and limited to anticoagulants and thrombolytic agents, which must be given immediately (at latest 3 hours) after having a stroke. Unfortunately, there is no effective neuroprotective medication available, which is able to stop the delayed degeneration of neurons following the initial stroke attack.
Stroke symptoms appear suddenly. The following acute symptoms can be observed: Sudden numbness of the face, arm or leg, difficulties in talking or understanding speech, trouble seeing in one or both eyes, sudden trouble in walking, loss of balance and coordination. Severe headache with no known cause does also occur. Even more importantly, there is a variety of severe disabilities occurring and persisting in post-stroke patients. Paralysis is a frequent disability resulting from stroke. Cognitive deficits (problems with thinking, awareness, attention and learning) are

also commonly observed. Post-stroke patients exhibit language deficits and also emotional deficits (like post-stroke depression). Furthermore, an uncommon type of pain, called central pain syndrome (CPS), can occur after having a stroke.

Currently the only effective treatment for thrombotic stroke is the use of anticoagulants (e.g. heparin), and thromobolytics (recombinant tissue plasminogen activator). Neuroprotective agents, which are effective in animal models, have generally proved ineffective in the clinic, and none are yet registered for use in stroke.

A number of experimental models have been developed for global ischemia and focal ischemia. The availability of these different models provides an opportunity to investigate mechanisms of stroke. Finding common features in different models or over several time points within one model should provide better insight into the mechanisms critical for stroke, and comparison of the models should help to understand development, progression and consequences of stroke.

Most common global ischemia models are

a) the two vessel model

Models of transient global ischemia resulting in patterns of selective neuronal vulnerability are models that attempt to mimic the pathophysiology of cardiac arrest or hemodynamic conditions that result from severe systemic hypotension. Reversible high-grade forebrain ischemia is generated by bilateral common carotid artery (CCA) occlusion. Together with systemic hypotension conditions it reduces the blood flow to severe ischemic levels (Smith et al. 1984, Acta Neuropathol. 64; p319-332). This model of transient global ischemia has the advantage of a one stage surgical preparation, the production of a high-grade forebrain ischemia and the possibility to conduct chronic survival studies in order to assess the potential of neuroprotective drugs.

b) the four vessel model

This model results in a high-grade forebrain ischemia but is produced in two stages, one to manipulate each of the CCA, and the second stage 24h later to produce the forebrain ischemia. The advantage is that the second step can be produced in awake freely moving animals (Pulsinelli et al. 1982, Ann.Neurol. 11; 491-498). Similar pathohistological results could be obtained for both vessel models.

c) the cardiac arrest model

Forebrain ischemia models are of value to study cerebral ischemia but these models do not exactly mimic the hemodynamic consequences of a cardiac arrest, which results in a complete ischemia of the brain, spinal cord and extracerebral organs (Katz et al. 1995; J. Cereb. Blood Flow Metab. 15; 1032-1039). The initial cardiac arrest models from Safar et al. (1982, Protection of tissue against hypoxia; Elsevier Biomedical Press; 147-170 ) or Korpaczew et al. (1982, Partol Fizjol Eksp Ter 3; 78-80), were developed further by Katz et al. (1989, Resuscitation 17; 39-53) and Pluta et al. (1991, Acta Neuropathologica 83; 1-11) to models with controllable insult. Katz and colleagues (1995) have reported a reproducible outcome model of cardiac arrest with apneic asphyxia of 8 min, leading to the cessation of circulation at 3-4 min of apnea and resulting in cardiac arrest of 4-5 min. At 72 hr after injury, widespread patterns of ischemic neurons were found in many brain regions, including cerebral cortex, caudate putamen CA1 and CA3 regions of hippocampus, thalamus, cerebellum and brain stem.

Pluta et al. described a primary mechanical cardiac arrest model whereby global ischemia was induced by cardiac arrest for 3 to 10 min with survival periods of the animals from 3 min to 7 days.

Although these models have several limitations, they provide a method for studying the mechanisms of neuronal injury resulting from the clinically realistic cerebral insult and screening potential cerebral resuscitation therapies.

Focal Ischemia models

Models of permanent or transient focal ischemia typically giving rise to localised brain infarction have routinely been used to investigate the pathophysiology of stroke. For example, models of middle cerebral artery (MCA) occlusion in a variety of species have gained increased acceptance due to their relevance to the human clinical setting.

a) the permanent MCA Occlusion models

Tamura and colleagues (1981, J. Cereb. Blood Flow Metab. 1; 53-60) developed a subtemporal approach as standard model of proximal MCA occlusion. In models of permanent MCA occlusion, electrocauterisation of the MCA proximal to the origin of the lateral lenticulostriate arteries is utilised routinely. In these models, severe reductions in blood flow are seen within the ischemic core, with milder reductions in blood flow within the border or penumbral regions. The addition of moderate arterial hypotension has the effect of enlarging infarct volume.

b) the transient MCA Occlusion models

In human ischemic stroke, recirculation frequently occurs after focal ischemia. Thus, models of transient MCA occlusions have also been developed, mainly in rats or mice, whereby surgical clip or sutures are introduced to induce a transient ischemic insult.

Some further animal models for stroke are considered in several reviews like the articles of W.D. Dietrich (1998; Int. Review of Neurobiology, Vol.42; 55-101) , Wiebers et al. (1990 Stroke 21; 1-3) or Zivin and Grotta (1990 Stroke 21; 981-983).

Object of the present invention is to provide a target for examination and development of a medicament for the treatment of stroke.

This object is met by a method for the development of a medicament for the treatment of stroke comprising the use of a polynucleotide sequence encoding Cathepsin Y or homologues or fragments thereof or the according polypeptide.

According to the invention it has been found that Cathepsin Y is differentially expressed under stroke, particularly under ischemic stroke. In the present invention it is shown that in the rat models Cathepsin Y expression is upregulated under stroke, particularly under global ischemia in the cardiac arrest model (Pluta et al, 1991 Acta Neuropathol. 83; 1-11).

The model is explained in more detail in the literature and in the examples below.

The term "polynucleotide sequence" or "nucleic acid sequence" designates in the present application any DNA or RNA sequence, independent of the length. Thus this term can describe short sequences like PCR primers or probes for hybridisation, as well as whole genes or cDNA of these genes.

The term "polypeptide" or "amino acid sequence" designates a chain of amino acids, independent of their length, however, in any case more than one amino acid.

As "homologues" of polynucleotide sequences such polynucleotide sequences are designated which encode the same type of protein as one of the polynucleotide sequences described herein. Accordingly as "homologues" of a polypeptide the polypeptides are designated, which have an amino acid sequence, wherein at least 70 %, preferably 80 %, more preferably 90 % of the amino acids are identical to one of the proteins of the present invention and wherein the replaced amino acids preferably are replaced by homologous amino acids. As "homologous" amino acids are designated those, which have similar features concerning hydrophobicity, charge, steric features etc.. Most preferred are amino acid sequences, containing the species- or family-dependent differences of the amino acid sequence. Particularly as "homologues" sequences are designated which correspond to one of the cited sequences in another species or individual. For example if in the present invention a rat model is used and the cited polynucleotide sequence encodes the rat protein, the according polynucleotide sequence and protein of a mouse in a mouse model is designated as "homologue". Further splice variants and members of gene families are designated as homologues.

"Fragments" of a polynucleotide sequence are all polynucleotide sequences, which have at least 10 identical base pairs compared to one of the polynucleotide sequences shown in the present application or by the genes represented by these polynucleotide sequences. The term "fragment" encloses therefore such fragments as primers for PCR, probes for hybridisation, DNA fragments included in DNA vectors like plasmids, cosmids, BACs or viral constructs, as well as shortened splice variants of the genes identified herein. As a fragment of a protein (polypeptide) amino acid sequences are designated which have at least three amino acids, preferably at least 10 amino acids. Therefore fragments serving as antigens or epitopes are enclosed in this designation.

In the present application the term "sequence" is used when either a polynucleotide sequence (= nucleic acid sequence) or a polypeptide (= amino acid sequence) or a protein is meant. That means, when it is irrelevant which type of sequence is used the type is not designated particularly, but with the more common term "sequence".

In the present application the term "stroke" means the development, occurence, progression and consequences of the disease state. Several features of the development, occurance and consequences of this disease are described herein above.

The basis of the methods and assays described in the present application is the examination of Cathepsin Y, which is differentially expressed under stroke. For the examination necessary for the development of any medicament each sequence can be used which allows the determination of the expression rate of the Cathepsin Y gene or the activity of the polypeptide / protein. The considered sequence can be the polynucleotide sequences SEQ ID NO. 1, SEQ ID NO. 2 or SEQ ID NO. 4, respectively or homologues or fragments thereof, as well as the polypeptides encoded thereby (SEQ ID NO. 3 or SEQ ID NO. 5, respectively) or homologues or fragments thereof.

According to the invention it has been found out, that the Cathepsin Y gene represented by the polynucleotide sequences SEQ ID No: 2 is differentially expressed in the cardiac arrest models for stroke, which is described above.

Therefore the present invention provides a target for the development of medicaments for the treatment of stroke, particularly for the treatment of ischemic stroke. Cathepsin Y has not yet been regarded in relation to stroke.

JP2000157263 describes a human Cathepsin Y useful in the elucidation and therapy for Alzheimer's disease,

neurodegenerative diseases, cancers and the like. Comparing the nucleic acid sequence shown in this publication with the sequences of common databases, however, shows that the provided sequence corresponds to Cathepsin F, not to Cathepsin Y.

WO 96/39194 teaches that the Cathepsin Y protein is involved in the secretion of β-amyloid peptide (β-AP) from cells, resulting in the development of Alzheimer's disease. The nucleic acid sequence as well as the protein sequence of Cathepsin Y is provided. For the treatment of Alzheimer's disease the inhibition of this protein is proposed, therefore inhibitors of Cathepsin Y are examined.

According to the present invention the Cathepsin Y sequences (polynucleotide sequence or polypeptide) can be used in any test system which allows the determination of either the expression rate of the gene or the activity of the polynucleotide sequence or of the polypeptide / protein.

For determination and comparison of the expression levels of at least one of the sequences identified in the present invention any of the commonly known methods can be used, either on RNA/cDNA level or on protein level. For example PCR, hybridisation, microarray based methods, western blot or 2-D protein gel analysis are suitable methods. One preferred method is the digital expression pattern display method (DEPD method), explained in detail in WO99/42610. The method used for determination of expression levels is not restrictive, as long as expressed amounts can be quantified.

Activity of the Cathepsin Y protein can be determined by contacting the protein with polypeptides under acid pH conditions and determining the concentration of free amino acids after cleavage. Activity of the Cathepsin Y protein is a carboxypeptidase activity. Methods for determination of Cathepsin Y activity are explained in detail in international patent application WO 96/39194.

Expression and activity of Cathepsin Y can further be determined in assay systems or models. Such assay systems may be in vivo, ex vivo or in vitro assays, for example a cellular assay system comprising cells expressing Cathepsin Y, or an assay comprising isolated Cathepsin Y.

In any assay or model at least one of the sequences is contacted with the compound(s) to be tested and samples are obtained, wherein expression levels or activity of the sequences are determined and compared to non-treatment conditions.

For examination of the expression rate of the gene, animal models can be used. As such a model any animal can be used wherein the necessary preparations can be carried out, however, mammalian models are preferred. Even more preferred are rodents, particularly preferred are rats and mice. The most preferred animal model of the present invention is a rat model.

Dependent of the model used, the samples can be derived from whole blood, cerebrospinal fluid (CSF) or whole tissue, from cell populations isolated from tissue, cerebrospinal fluid (CSF) or blood or from single cell populations (i.e. cell lines).

In one embodiment of the invention cellular assays can be used. Preferred cells for cellular assays are eukaryotic cells, more preferably mammalian cells. Most preferred are neuronal-like cells, like SHSY5Y (neuroblastoma cell line), hippocampal murine HT-22 cells, primary cultures from astrocytes, cerebral cortical neuronal-astrocytic co-cultures, mixed neuronal/glial hippocampal cultures, cerebellar granular neuronal cell cultures, primary neuronal cultures derived from rat cortex (E15-17), or COS cells (African green monkey, kidney cells); CHO cells (Chinese hamster ovary), or HEK-293 cells (human embryonic kidney).

The Cathepsin Y sequences of the present invention can be used for diagnosing stroke, particularly ischemic stroke of a human outside of the living body, by determining the expression levels or activity of Cathepsin Y sequences in comparison to the non-disease status. During treatment period of a patient the expression or activity of the sequences can also be used for assessing the efficacy of stroke treatment outside of the body. In these cases blood, CSF or tissue is removed from the patient and expression or activity is determined in the samples.

The disease status, which is considered in the present invention, is stroke. The preferred types of stroke are ischemic and hemorrhagic stroke. *Further diseases, which might be related to stroke, are severe headache, paralysis, cognitive deficits, speech problems, emotional difficulties, daily living problems, and central pain syndrome.*

Independent whether the efficacy of stroke treatment or the efficiency of a compound for the treatment of stroke shall be examined or a stroke status is diagnosed or characterised, determination of the expression level or the activity of Cathepsin Y is carried out outside of a living body. As mentioned above, detection of the expression of Cathepsin Y can be carried out by any method known in the art. The method of detection is not limiting the invention.

Expression levels can be detected either on basis of a polynucleotide sequence or by detecting the according polypeptide, encoded by said polynucleotide sequence.

Preferred methods for detection and determination of the gene expression levels are PCR of cDNA, generated by reverse transcription of expressed mRNA, hybridisation of polynucleotides (Northern-, Southern Blot systems, *In situ* hybridisation), DNA-microarray based technologies, detection of the according peptides or protein via e.g. Western Blot systems, 2-dimensional gel analysis, protein microarray based technologies or quantitative assays like e.g. ELISA tests.

The most preferred method for quantitative analysis of the expression levels is the differential expression pattern display method (DEPD), described in detail in WO99/42610.

By using any of the Cathepsin Y sequences (polynucleotide sequence or polypeptide / protein) the efficiency of compounds for reducing the expression of the sequence or the activity of the protein can be tested. Therefore the sequences of the present invention can be used for identifying therapeutic agents and their efficacy for the treatment of stroke.

For example a method can be used comprising:

a) providing a test cell population comprising cells capable of expressing the Cathepsin Y gene(s) or homologues or fragments thereof
b) contacting said test cell population with the test therapeutic agent,
c) detecting the expression of the Cathepsin Y gene(s) in said test cell population,
d) comparing the expression of the gene(s) in the test cell population to the expression of the gene(s) in a reference cell population whose disease stage is known; and
e) identifying a difference in expression levels of the considered sequences, if present, in the test cell population and the reference cell population, thereby identifying a therapeutic agent for treating stroke.

For this method an animal model can be used or test cells can be obtained from a subject, an animal model or cell cultures of fresh cells or cell lines. Further *in vitro* assays may be used.

Agents or compounds, which have any influence on the expression rate of the Cathepsin Y gene(s) might be used as a medicament for the treatment of stroke. A method examining the expression rate of the Cathepsin Y gene can therefore be used for testing agents and compounds for their efficiency for treatment of stroke. Which model is used is not relevant, as long as the model allows determining differences in expression amounts.

In such a model cells are contacted with the interesting agent or compound, and expression of Cathepsin Y gene is determined in comparison to the expression in cells which have never been contacted by the according agent /compound. Contacting the cells with the agent / compound can either be achieved by administering the agent / compound to an animal, or to isolated cells of tissue or blood, or cell lines in culture.

By examination of the influence the considered agent / compound has to the expression of the Cathepsin Y gene the efficacy of the agent / compound for treating stroke can be estimated. This allows the decision whether it is worthwhile to develop a medicament containing such an agent or compound for the treatment of stroke.

Whether the expression is determined on the basis of mRNA generation (transcription level) or on basis of protein generation (translation level) is not relevant, as long as the difference of the expression rate can be determined. Therefore the polynucleotide sequence, as well as the polypeptide or protein shown in the present application can be used for the development of a medicament.

The development of a medicament can be desirable for example if the considered compound /agent has any influence on the regulation of the expression rate or on the activity of Cathepsin Y.

Said influence of a compound or agent can be examined by a method comprising contacting a sample comprising the nucleic acid sequence or homologues or fragment thereof or the according polypeptides of the present invention with a compound that binds to said sequence in an amount sufficient to determine whether said compound modulates the expression rate or the activity of the polynucleotide or polypeptide sequence.

By such a method a compound or agent can be determined modulating the expression rate or activity of the nucleic acid sequence or homologues or fragments thereof or the according polypeptides.

A method for determination of the efficiency of a compound / agent for the treatment of stroke by determination of the activity of the Cathepsin Y protein comprises for example contacting the Cathepsin Y protein with a compound /agent in presence of a known polypeptide which is a target of the Cathepsin Y, determining the amino-terminal amino acid of the peptide or the amount of free amino acids in the sample after incubation, comparing the amino-terminal amino acid of the peptide or the amount of free amino acids with the result in a sample which doesn't contain the compound /agent.

If a compound / agent is able to decrease or inhibit the activity of the Cathepsin Y protein it might be useful as a medicament for the treatment of stroke.

Examples for compounds, which can decrease Cathepsin Y activity, are compounds of formula I:

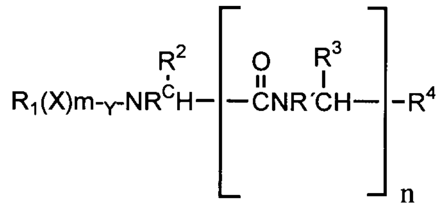

formula I

wherein:

R is selected from the group consisting of hydrogen, alkyl of from 1 to 6 carbon atoms, and where R and R2 are joined to form a ring structure of from 4 to 10 carbon atoms,

R' is selected from the group consisting hydrogen, alkyl of from 1 to 6 carbon atoms and where R' and $R^3$ are joined to form a ring structure of from 4 to 10 carbon atoms,

$R^1$ is selected from the group consisting of alkyl of from 1 to 4 carbon atoms substituted with from 1 to 5 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur wherein said substituted alkyl group is optionally further substituted with from 1 to 2 hydroxyl groups, alkenyl of from 2 to 4 carbon atoms substituted with from 1 to 4 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur, aryl of from 6 to 10 carbon atoms, aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, fluorenyl, heterocycles of from 3 to 14 carbon atoms having from 1 to 3 heteroatoms selected from the group consisting of nitrogen, oxygen and sulfur;

$R^2$ and $R^3$ are independently D- or L-amino acid side chains of at least 2 carbon atoms with the proviso that said amino acid side chains do not include the proline side chain;

$R^4$ is selected from the group consisting of -C(O)CH =N=N, $-CH_2OH$, -C=NOH, and $-C(O)R^5$ where $R^5$ is hydrogen, alkyl of from 1 to 6 carbon atoms, haloalkyl of from 1 to 6 carbon atoms and 1 to 2 halo groups, alkoxy of from 1 to 6 carbon atoms, $-NR^6R^7$ where $R^6$ and $R^7$ are independently selected from the group consisting of hydrogen and alkyl of from 1 to 6 carbon atoms, and aryl of from 6 to 10 carbon atoms, and $-N(CH_3)OCH_3$;

X is selected from the group consisting of -0-, $-NR^9-$, and -S- where $R^9$ is selected from the group consisting of hydrogen, alkyl of from 1 to 6 carbon atoms and aryl of from 6 to 10 carbon atoms;

Y is selected from the group consisting of -C(O)- and -C(S)-;

m is equal to zero or one; and

n is equal to zero, one or two,

or pharmaceutically acceptable salts thereof

with the proviso that when $R^1$ is 1-naphthyl $R^2$ is $-CH(CH_3)_2$ (L-isomer), $R^3$ is $-CH_2$-Ø (L-isomer), Y is -C(O)-, m is zero and n is one, then $R^4$ is not $-N(CH_3)OCH_3$, with the further proviso that when R' is diphenylmethyl,

$R^2$ is p-(benzyloxy)benzyl (L-isomer), Y is -C(O)-, and m and n are zero, then

$R^4$ is not $-N(CH_3)OCH_3$, and with still the further proviso that when $R^1$ is (1,2diphenyl)ethenyl, Y is -C(O)-, $R^2$ is $-CH_2$-Ø (L-isomer), and m and n are zero, then $R^4$ is not $-N((H_3)OCH_3$.

In a preferred embodiment

$R^1$ includes benzyl, trityl, diphenylmethyl, 4phenylbutyl, 2-phenylethyl, naphthyl, pyridyl, fluorenyl, xanthanilyl, and the like.

$R^2$ and $R^3$ are independent side chains of a D- or L- amino acid having at least 2 carbon atoms with the proviso that $R^2$ and $R^3$ are not proline. Such side chains refer to the $R^8$ substituent found on naturally occurring and synthetic amino acids of the formula $H_2NCHR^8COOH$. Side chains of naturally occurring amino acids include, by way of example only, those where $R^8$ is the L-isomer of $(CH_3)^2CH-$ (valine), $(CH_3)_2CHCH_2$ (leucine), $CH_3CH_2CH(CH_3)$-(isoleucine), $ØCH_2$-(phenylalanine), (3-indolyl)-$CH_2$- (tryptophan), $CH_3SCH_2CH_2$- (methionine), $CH_3CH(OH)$ (threonine), p-HO-Ø-$CH_2$-

(tyrosine), $H_2NC(O)CH_2$- (asparagine), $H_2NC(O)CH_2CH_2$- (glutamine), $HOC(O)CH_2$- (aspartic acid), $HOC(O)CH_2CH_2$- (glutamic acid), $H_2NCH_2CH_2CH_2CH_2$- (lysine), $H_2NC(NH)NHCH_2CH_2CH_2$- (arginine), 4-imidazolyl-$CH_2$- (histidine) and the like.

Side chains of synthetic amino acids include the D-isomer of the above noted naturally occurring amino acids as well as those where $R^8$ is selected from the group consisting of alkyl of from 2 to 6 carbon atoms (where the alkyl group does not occur in naturally occurring amino acids), cycloalkyl of from 3 to 8 carbon atoms, and alkyl of from 1 to 6 carbon atoms substituted with from 1 to 2 substituents selected from the group consisting of aryl of from 6 to 10 carbon atoms,
aryl of from 6 to 10 carbon atoms substituted with from 1 to 3 substituents selected from the group consisting of alkyl of from 1 to 6 carbon atoms, alkoxy of from 1 to 6 carbon atoms, aryl of from 6 to 10 carbon atoms, and aryloxy of from 6 to 10 carbon atoms, and heteroaryl of from 3 to 14 carbon atoms having from 1 to 3 heteroatoms selected from the group consisting of nitrogen, oxygen and sulfur (where the substituted alkyl group does not occur in naturally occurring amino acids).

Particularly preferred amino acid side chains include the D- and L-isomers of valine, leucine, phenylalanine, tryptophan and isoleucine.

$R^4$ preferably is -CH=N=N or -C(O)H.

X preferably is -O-.

Y preferably is -C(O)-.

m and n preferably are 0 or 1.

In the present application -Ø means a phenyl residue.

Preferred compounds include, by way of example, the following compounds as defined by formula II below, including all isomers thereof, wherein the amino acid side chain for $R^2$ and $R^3$ is indicated beneath the $R^2$ and $R^3$ substituent:

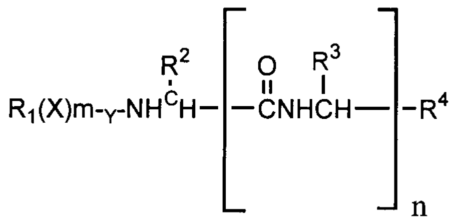

formula II

| R | 'X | m | Y | $R^2$ | n | $R^3$ | $R^4$ |
|---|---|---|---|---|---|---|---|
| Ø-$CH_2$- | 0 | 1 | -C(O)- | -$CH(CH_3)_2$ (valine) | 1 | -$CH_2$-Ø (phenylalanine) | -C(O)CH=N=N |
| Ø-$CH_2$- | 0 | 1 | -C(O)- | -$CH_2$-Ø (phenylalanine) | 1 | -$CH_2$-Ø (phenylalani ne) | -C(O)CH=N=N |
| $(Ø)_2$-CH- | - | 0 | -C(O)- | -$CH_2$-Ø (phenylalanine) | 0 | - | -C(O)-H |
| Ø-$(CH_2)_4$- | - | 0 | -C(O)- | -$CH_2$-Ø (phenylalanine) | 0 | - | -C(O)-H |
| $(Ø)_3$-C- | - | 0 | -C(O)- | -$CH_2$-Ø (phenylalanine) | 0 | - | -C(O)-H |
| $(Ø)_2$-CH- | - | 0 | -C(O)- | -$CH_2$-$CH_2$-Ø (homophenylalanine) | 0 | - | -C(O)-H |
| ØCH=C(Ø) | - | 0 | -C(O)- | -$CH_2$-Ø (phenylalanine) | 0 | - | -C(O)-H |
| $(Ø)_2$-CH- | - | 0 | -C(O)- | -$CH_2$-(3-indolyl) | 0 | - | -C(O)-H |
| Ø-$CH_2$- | 0 | 1 | -C(O)- | -$CH_2$-Ø (phenylalanine) | 1 | -$CH(CH_3)_2$ (valine) | -C(O)-H |
| Ø-$CH_2$- | 0 | 1 | -C(O)- | -$CH(CH_3)_2$ (valine) | 1 | -$CH_2$-$CH(CH_3)_2$ (leucine) | -C(O)-H |

The term "heterocycles containing from 3 to 14 carbon atoms and 1 to 3 hetereoatoms selected from the group consisting of nitrogen, oxygen and sulfur" refers to saturated and unsaturated heterocyclic groups having the requisite number of carbon atoms and heteroatoms. Suitable heterocyclic groups include, by way of example, furazanyl, furyl, imidazolidinyl, imidazolyl, imidazolinyl, indolyl, isothiazolyl, isoxazolyl, morpholinyl (e.g. morpholino), oxazolyl,

piperazinyl (e.g. 1-piperazinyl), piperidyl (e.g. 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g. 1-pyrrolidinyl), pyrrolinyl, pyrrolyl, quinoxalinyl, thiadiazolyl, thiazolyl, thienyl, thiomorpholinyl (e.g. thiomorpholino), triazolyl, and xanthanilyl.

If in the present application a chemical group is described as containing for example "from 1 to 6 carbon atoms" or "from 6 to 10 carbon atoms" or "from 1 to 4 heteroatoms" it is meant that the group can contain either 1 or 2 or 3 or 4 or 5 or 6, or 6 or 7 or 8 or 9 or 10, or 1 or 2 or 3 or 4 of said atoms, respectively.

Heterocyclic groups can be substituted or unsubstituted. Where the heterocyclic group is substituted, the substituents are selected from alkyl of from 1 to 6 carbon atoms, alkoxy of from 1 to 6 carbon atoms, aryl of from 6 to 10 carbon atoms, aryloxy of from 6 to 10 carbon atoms, and halo.

Preferred heterocycles include well known cyclic aromatic groups containing heteroatoms within the cyclic structure. Such groups include, by way of example, furyl, imidazolyl, oxazolyl, pyrazolyl, pyridyl, pyrimidinyl, thiazolyl, and triazolyl.

The term "alkyl" refers to straight and branched chain alkyl groups such as methyl, ethyl, n-propyl, iso-propyl, n-butyl, tert-butyl, sec-butyl, isobutyl, n-pentyl, n-hexyl, 2-methylpentyl, and the like; whereas the term "alkoxy" refers to -0-alkyl substituents.

The term "aryl" refers to aromatic substituents comprising carbon and hydrogen such as phenyl, naphthyl and the like whereas the term aryloxy refers to -0-aryl substituents where aryl is as defined above.

The term "halo" or "halogen" refers to fluorine, chlorine, bromine and iodine and preferably fluorine and chlorine.

The term "pharmaceutically acceptable salts" refers to the non-toxic alkali metal, alkaline earth metal, and ammonium salts commonly used in the pharmaceutical industry including the sodium, potassium, lithium, calcium, magnesium, barium, ammonium, and protamine zinc salts, which are prepared by methods well known in the art. The term also includes non-toxic acid addition salts, which are generally prepared by reacting the compounds of this invention with a suitable organic or inorganic acid.

Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, and napsylate salts, and the like. The particular salt employed is not critical.

Particularly preferred embodiments of the compounds usable as a medicament for the treatment of stroke by inhibiting protein activity of Cathepsin Y are the compounds 1 to 13 as shown in the following:

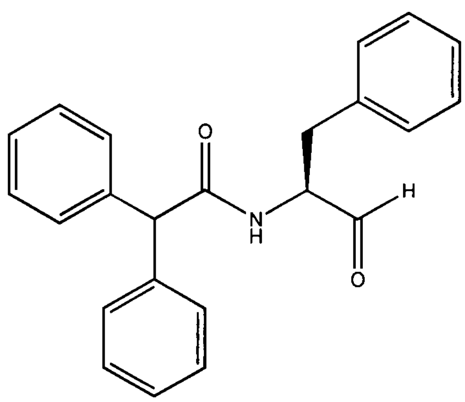

Compound 1

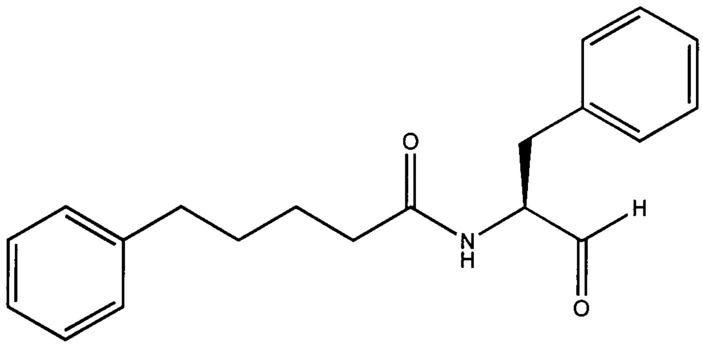

Compound 2

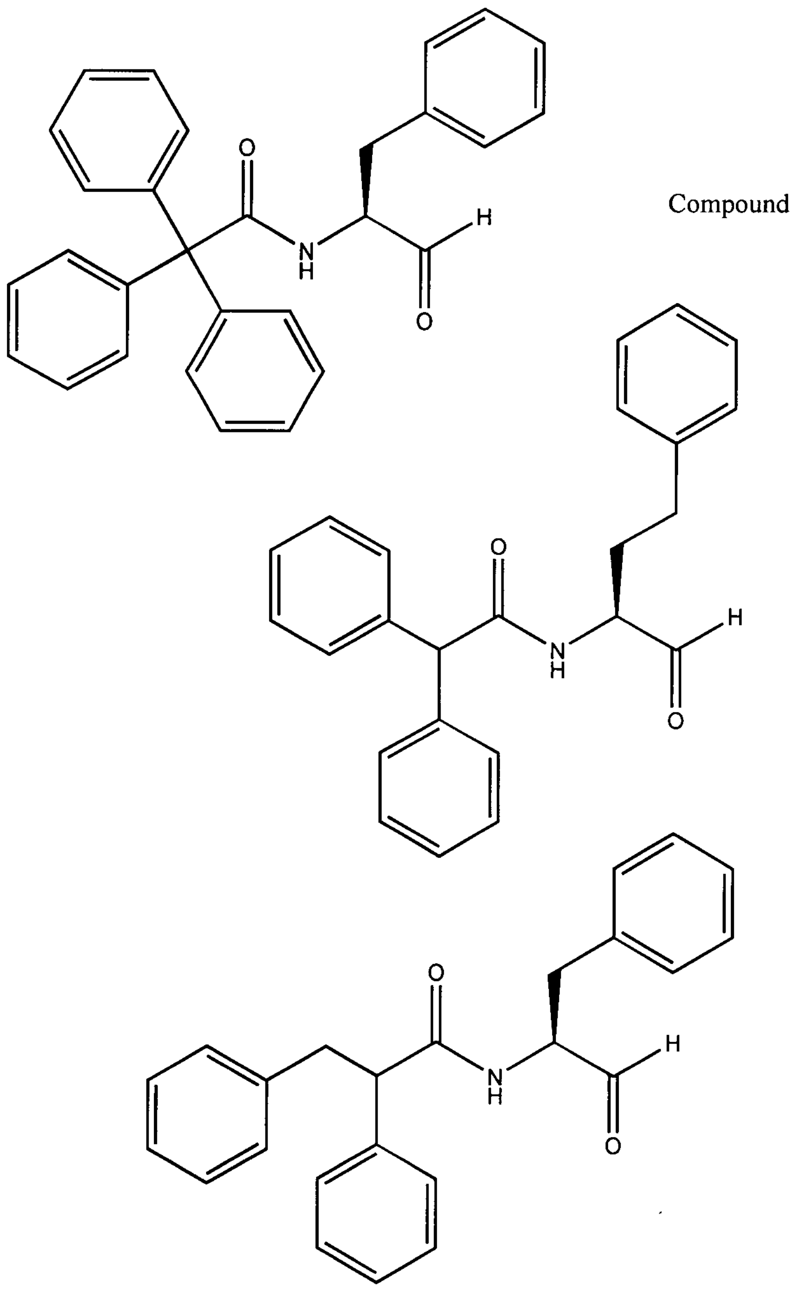

Compound 3

Compound 4

Compound 5

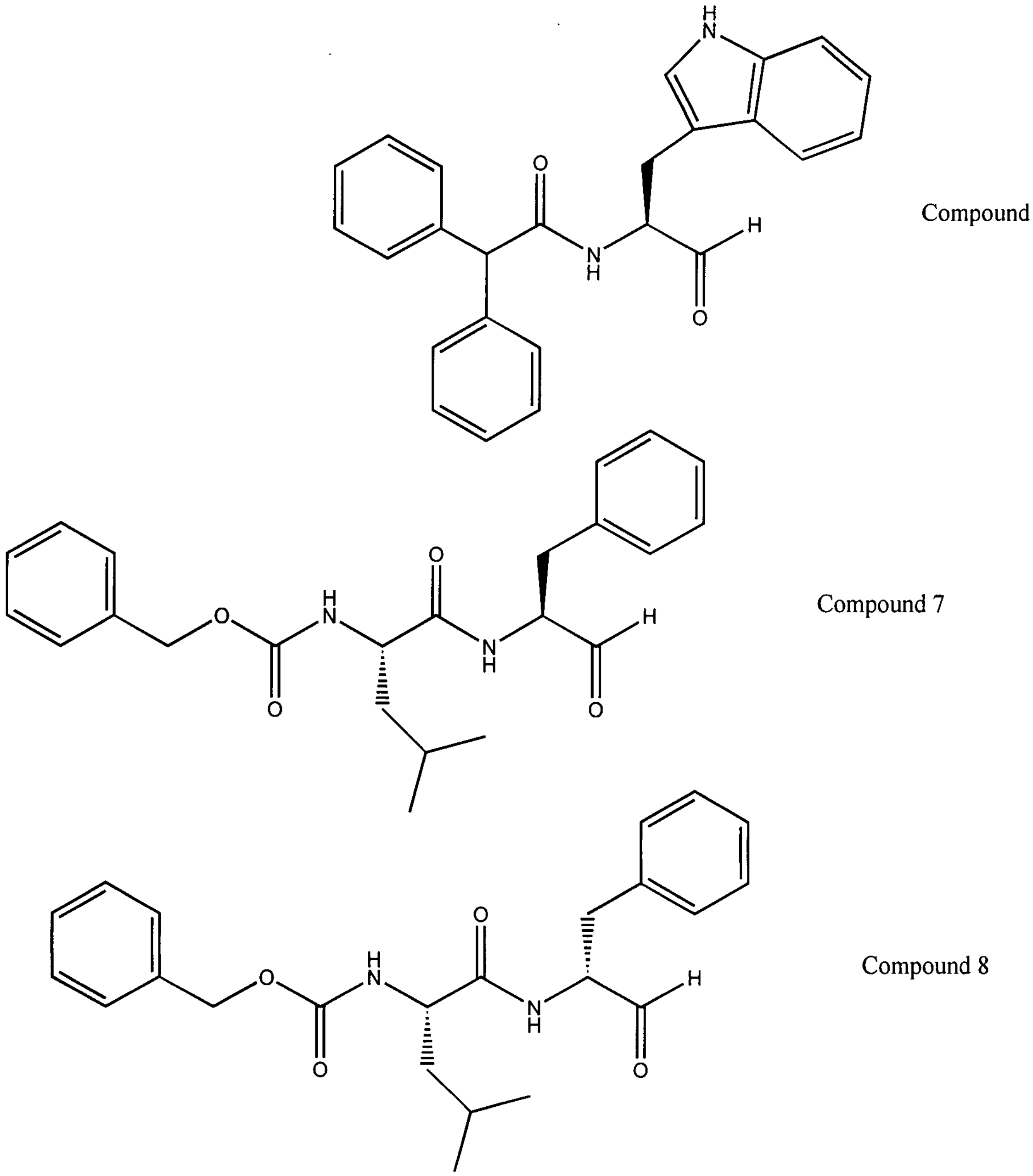

Compound 6

Compound 7

Compound 8

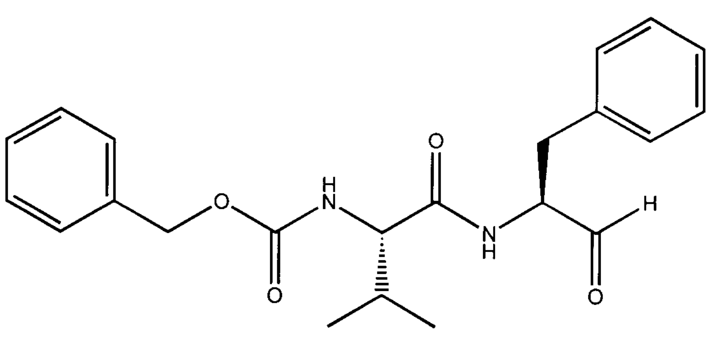

Compound 9

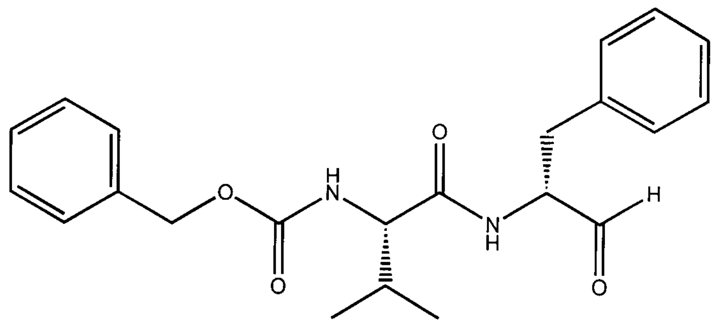

Compound 10

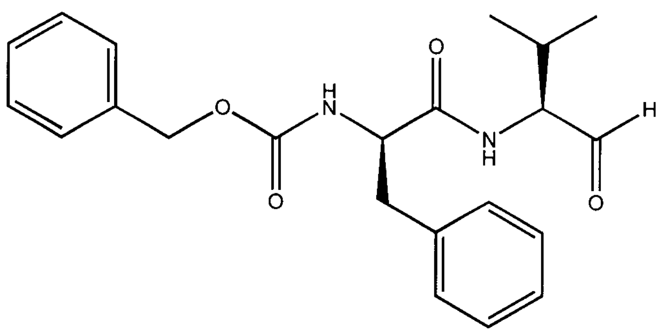

Compound 11

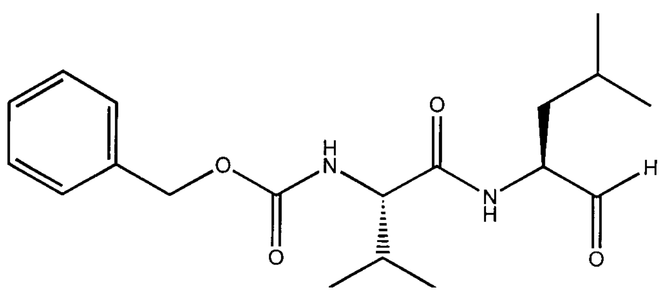

Compound 12

Preparation of the compounds 1 to 12 of the present application is explained in detail in WO96/39194.

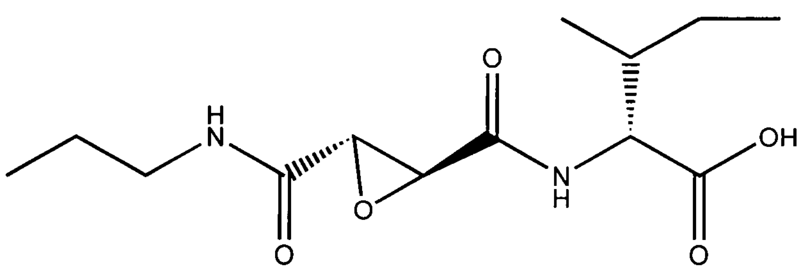

Compound 13

Preparation of compound 13 is explained in detail in the article of Therrien, C. et al., Biochemistry (2001) 40, 2702-2711.

Furthermore the sequences of the present invention itself can be used as a medicament.

An example for such a use is the use of a polynucleotide sequence as an antisense agent. Antisense agents, including, but not limited to, ribonucleotide or desoxyribonucleotide oligomers, or base-modified oligomers like phosphothioates, methylated nucleotides, or PNAs (peptide nucleic acids), can hybridise to DNA or mRNA, inhibiting or decreasing transcription or translation, respectively. Thus, polynucleotide sequences of a gene, which is increased in expression rate under stroke, can be used as antisense agents to decrease the expression rates of said gene. Further such polynucleotide sequences can be used for gene therapy.

A pharmaceutical composition comprising a polynucleotide sequence according to the present invention can be any composition, which can serve as a pharmaceutical one. Salts or aids for stabilising the sequence in the composition preferably are present.

For the determination of the expression of Cathepsin Y the generated sequence has to be detected. Therefore several reagents can be used, which are for example specific radioactive or non-radioactive (e.g. biotinylated or fluorescent) probes to detect nucleic acid sequences by hybridisation, primer sets for the detection of one or several of the nucleic acid sequences by PCR, DNA microarrays, antibodies against one of the polypeptides, or epitopes, or antibody or protein microarrays. Such reagents can be combined in a kit, which can be sold for carrying out any of the described methods.

For further examinations or experiments it might be desirable to include the nucleic acid sequence of the present invention into a vector or a host cell. By including the sequence in a host cell, for example cellular assays can be developed, wherein the gene, polynucleotide sequences and the according protein / polypeptides can be used or examined.

Further the sequences defined in the present invention can be used to "design" new transgenic animals as models for stroke. Therefore the animals are "created" by manipulating the gene(s) encoding Cathepsin Y in a way that their expression in the transgenic animal differs from the expression of the same gene(s) in the wild type. "Manipulation" preferably results in a different expression level of the Cathepsin Y in the transgenic animal compared to the wild type, or in a protein with different enzymatic activity. Whether the expression level or the protein activity becomes higher or lower than the wild type under the same conditions depends on the desired model for stroke. Methods of gene manipulation and methods for the preparation of transgenic animals are commonly known to those skilled in the art.

Figures:

Figure 1 shows Cathepsin Y transcript differentially expressed over several time points in the cardiac arrest model. Cathepsin Y is examined in its expression level over a time period of 0.5 hrs after cardiac arrest to 2 years after surgery and compared to sham operated controls. Cathepsin Y is described as differentially expressed when the sequence is up or down regulated at one or more time points with a certain statistical relevant significance value ($\Delta h \geq \pm 0.2$).

X-axis describes the time points analysed by DEPD, 1= 0.5h post operation, 2= 1 hr post operation, 3= 3 hrs post operation, 4= 6hrs post operation, 5=day 3 post operation, 6=day 7 post operation, 7= 2 years post operation. The Y-axis shows $\Delta$ h, which represents the normalised difference of expression (peak height) of Cathepsin Y between a control group and a treated group. x-fold difference in gene expression is calculated by

$$\frac{1+\Delta h}{1-\Delta h}$$

0= no change to control, + = up regulation, - = down regulation; (0,2 = 1,5 fold; 0,3 = 1,86 fold; 0,4 = 2,33 fold; 0,5 = 3 fold expression level).

The following examples are provided for illustration and are not intended to limit the invention to the specific example provided.

Examples:

Example 1: Preparation of rat cardiac arrest model

Cardiac arrest was performed in female Lewis-rats at 3 months of age (150-220g), resulting in total cessation of blood flow leading to global cerebral ischemia. After 10 mm of ischemia, the animals were resuscitated by external heart massage and ventilation. The group size was 2 x 3 animals.

For induction of cardiac arrest, a special blunt-end, hook-like probing device was inserted into the right parasternal line across the third intracostal spaces into the chest cavity. Next, the probe was gently pushed down the vertebral column until a slight resistance from the presence of the right pulmonary veins was detected. The probe then was tilted 10-20° caudally. Then, the probe was rotated in a counter-clockwise direction about 135-140° under the inferior vena cava. At this position, the occluding part of the device was positioned right under the heart vessle bundle (inferior vena cava, superior right and left vena cava, ascending aorta, and pulmonary trunk). The pulmonary veins (left and right) were closed by the rotation of the occluding part of the hook. In the last step, the probe was pulled up with concomitant compression of the heart vessel bundle against the sternum. The end of the occluding portion of the hook was then positioned in the left parasternal line in the second intercostal space. To prevent upward movement of the chest and to insure complete ligation of the vessels, simple finger pressure was applied downward the sternum, producing total hemostasis and subsequent ventricular arrest. The effect of the whole procedure is total cessation of both arterial and venous blood flow, it essentially represents the onset of clinical death. After 2,5-3,5 min, the probe was released and removed from the chest by reverse procedural succession, and the animals remained in this position until the beginning of resuscitation.

The resuscitation procedure consisted of external heart massage until spontaneous heart function was recovered and controlled respiration occured. During this time, air was pumped through a polyethylene tube inserted intratracheally that was connected to a respirator. External heart massage was produced by the the index and middle fingers rapidly striking against the chest (sternum) for 1-2 min at the level of the fourth intracostal area with a frequency of 150-240/min in continuous succession. The ratio of strikes to frequency of ventilation was 6:1 or 8:1.

An electrocardiogramm (lead II-EEG) was recorded continuously during the course of the experiment. Moreover, heart activity was monitored using a loud speaker connected to the output lead of the electroencephalograph. Additionally, the cranial bones were exposed at the sagittal and coronal sutures, where silver-needle electrodes were attached for recording on an electrocorticogramm (ECoG). All measurements were registered on a ten-channel electroencephalogramm (Accutrace-100A, Beckmann).

2 x 3 sham operated animals served as controls. These animals were treated similarly to the experimental group with one major exception. Under anesthesia, the probe was inserted through the chest wall into the plural cavity as has already been described above but without further manipulation and torsion of the probe. The probe remained in the chest for essentially the same time period (3,5 min) as in the experimental group. The control animals were then returned to their cages for recovery.

Tissue preparation occurred 0.5h, 1h, 3hrs, 6hrs, 3 days, 7 days and 2 years after surgery. Tissues were frozen on liquid nitrogen prior to RNA preparation.

Example 2: Determination of expression levels

Gene expression profiling by DEPD-analysis starts with the isolation of 5-10µg total RNA. In a second step, double-stranded cDNA is synthesized. Through an enzymatic digest of the cDNA with three different type IIS restriction enzymes, three pools with short DNA-fragments containing single-stranded overhangs are generated. Afterwards, specific DNA-adaptor-molecules are ligated and in two subsequent steps 3.072 PCR reactions are performed by using 1024 different unlabelled 5'primer and a common FAM fluorescent labelled 3'-primer in the last PCR step. Subsequently, the 3072 PCR pools are analysed on an automatic capillary electrophoresis sequencer.

Differential gene expression pattern of single fragments are determined by comparison of normalized chromatogram peaks from the control groups and corresponding operated animals.

Example 3: Sequencing and Databank analysis of the obtained sequences

Differentially expressed peaks are confirmed on polyacrylamid gels by using radioactive labelled 3' primer instead of the FAM fluorescent primer. Differentially expressed bands are cut from the gel. After a short elution step in 60µl 10mM Tris pH8, fragments are re-amplified by PCR using the same primer as used in the DEPD analysis. Resulting PCR products are treated with a mixture of Exonuclease I and shrimp alkaline phosphatase prior to direct sequencing. Sequencing reactions are performed by using a sequencing kit like DYE-namic ET dye terminator sequencing kit, and subsequently analysed by capillary electrophoresis (Megabace 1000, Amersham).

Prior to a BLAST analysis (Altschul et al. 1997, Nucleic Acids Res. 25:3389-3402) against Genbank (Release 126), sequence was quality verified and redundant sequences or repetitive motifs are masked.
Results are shown in Table 1 and are compared to human sequence.

| SEQ ID NO: | name | length |
|---|---|---|
| 1 | EST of Cathepsin Y (rat) | 255 bp |

(continued)

| SEQ ID NO: | name | length |
|---|---|---|
| 2 | rattus norvegicus Cathepsin Y DNA | 1387 bp |
| 3 | rat Cathepsin Y protein | 306 aa |
| 4 | human Cathepsin Y DNA | 1500 bp |
| 5 | human Cathepsin Y protein | 303 aa |

Expression of the Cathepsin Y gene in the stroke model over a time period of 0.5 h to 2 years is shown in figure 1.

Example 4: Enzymatic activity of Cathepsin Y protein

Cathepsin Y does not have the standard endopeptidic activity associated with proteases. In an effort to identify whether the enzyme has other proteolytic activity, a number of randomly selected synthetic oligopeptides were incubated with purified Cathepsin Y at pH 5.5 or 4.5. Specifically, purified Cathepsin Y was incubated with the selected synthetic oligopeptides (50 μg/ml) at either pH 4.5 or pH 5.5, for 1 hour at 37°C. Samples were quenched by the addition of trifluoroacetic acid to 1% final concentration, then analysed by reverse phase HPLC on a Vydac C18 column, using a gradient of increasing acetonitrile in 0.1% trifluoroacetic acid. Individual peaks (parent and new product(s)) were collected, then analysed by acid hydrolysis followed by amino-acid analysis.
The results are summarised below:

| Parent Sequence | Product(s) |
|---|---|
| LFYDQSPTATI | LFYDQSPTAT(aa 1-10 of the parent sequence)<br>LFYDQSPTA (aa 1-9 of the parent sequence)<br>LFYDQSPT (aa 1-8 of the parent sequence) |
| YKRDMVGGVVIA | YKRDMVGGVVI (aa 1-11 of the parent sequence)<br>YKRDMVGGVV (aa 1-10 of the parent sequence) |
| EGYYGNYGVYA | EGYYGNYGVY (aa 1-10 of the parent sequence)<br>EGYYGNYGV (aa 1-9 of the parent sequence) |
| FFDEPNPGVTIY | FFDEPNPGVT (aa 1-10 of the parent sequence) |

The above peptides, as well as other peptides recited herein, are listed, per convention, from amino terminus to the carboxyl terminus.
The results from a number of such experiments (all not shown) suggest that the proteolytic activity of Cathepsin Y is a sequential removal of the carboxy terminal amino acids, which is direct evidence for carboxypeptidase activity. No evidence of any endopeptidase or aminopeptidase activity was seen with any of these substrates. These data strongly suggest that the predominant proteolytic activity manifested by Cathepsin Y is carboxypeptidase activity.
The quantitative analysis of carboxypeptidase activity of Cathepsin Y is based on the detection of the new free amino-terminus generated on cleavage of a selected substrate. This is accomplished by reacting with the reagent ophthalaldehyde in an alkaline solution in the presence of 2-mercaptoethanol (Simons, et al., JACS, 98:7098-7099, (1976)). The peptide EGYYGNYGV was synthesised acetylated on its amino-terminus so that on cleavage by Cathepsin Y, the only free amino-terminus present in the reaction mixture will be that of the valine residue, cleaved off by the carboxypeptidase activity of the protease.
A standard curve was constructed by incubating varying concentrations of valine (0-20 μM) in 0.25 M sodium borate, pH 10, containing 0.05% 2-mercaptoethanol and 60 μg/ml o-phthalaldehyde, in individual wells of a 96 well microtiter plate. The resulting fluorescence is read in a plate reading Cytofluor (ex 340, em 460 nm). There is a linear increase in the signal proportional to the amount of free valine present.
In order to determine the pH optimum of the enzyme, reaction mixtures were set up with enzyme and substrate (0.2 mg/ml) in a total reaction volume of 0.1 ml) in individual wells of 96-well microtiter plates, in 20 mM sodium acetate buffers at different pHs, with 0.1 % 2-mercaptoethanol present. Control wells were set identically except for the presence of enzyme. At various timepoints after incubation at room temperature, the reactions were quenched by the addition

of an equal volume of 0.45 M sodium borate, pH 10, with 0.25 mg/ml o-phthalaldehyde, and the fluorescence measured. In the absence of added enzyme, no measurable fluorescence is generated above background, even with extended incubations. In the presence of enzyme, there is a time-dependent increase in fluorescence. Based on this analysis, the pH optimum for carboxypeptidase activity was determined to be about 4.5, with the activity dropping off at both lower and higher pHs.

Using this assay at pH 4.5, the ability of a number of compounds to inhibit the activity of Cathepsin Y was tested, by adding the desired concentration of the inhibitor in the incubation mixture along with enzyme and substrate, and measuring the decrease in fluorescence (if any) relative to enzyme control. Each of the compounds 1 to 13 tested in this analysis indicated inhibition of Cathepsin Y activity.

<110> Biofrontera Pharmaceuticals AG

<120> Cathepsin Y for the development of a medicament for the treatment of stroke

<130> 10662EPalleSeq

<140>
<141>

<160> 5

<170> PatentIn Ver. 2.1

<210> 1
<211> 255
<212> DNA
<213> rattus norvegicus

<400> 1
gggaggagcg accgacacga actagaaatt atgagcacaa gtacactgga atcctccagc 60
ttcagagctg cttcctccac ccacagacct gcttcctcct ccaccgtgcc cgagcaggcc 120
taacctccag accgtcagag aggacagcta tggtctagga cagttctggt gttaccctgg 180
agtccacggg aggggaacta gtccagactg cctgagatga gtaaagtatc tggcgtcacc 240
aaaaaaaaaa aaaaa 255

<210> 2
<211> 1387
<212> DNA
<213> rattus norvegicus

<400> 2
ctcactttat ctggggtgcg gatcgggtca agaggttgaa ggtgctgtgc gtgatccagg 60
atccaagttg gcccccggag caggagcatg gcgtcgtcgg gatcggtgca gcagctgcgg 120
ctggtgctgc tgatgttgct gctggccggt gcggcacggg ccagcctcta cttccgcccg 180
ggccagacct gctaccgacc ccttcacagg gaccacctgg ctctgctggg gcgcaggacc 240
tatcctcggc cacatgagta cctgtcccca gcggatctcc ccaagaactg ggactggagg 300
aatgtgaacg gtgtcaacta tgccagcgtc acgaggaatc agcatatccc acagtactgt 360
ggttcttgct gggcccacgg cagcaccagt gccttggcag atcgcatcaa catcaagaga 420
aaaggtgcat ggccctccac cctgttgtcg gtgcagaatg tcatcgactg cggcaatgct 480
ggttcttgtg agggggcaa tgaccttccg gtgtgggagt atgcccacaa gcatggcatc 540
cccgatgaga cctgcaacaa ctaccaggcc aaggaccaag aatgtgacaa gtttaaccag 600
tgtgggacct gcactgaatt caaagagtgc cacaccatcc agaattatac cctctggaga 660
gtgggtgact atggctccct gtccgggagg gagaagatga tggcagagat ctacgccaat 720
ggtcccatca gctgcgggat aatggcaacc gagaggatgt ctaactacac tggaggcatc 780
tatactgagt accagaacca ggccattatc aaccacatca tctctgtagc cggctggggt 840
gtcagcaatg atggcatcga gtattggatt gtccgcaatt catggggcga gccctggggt 900

```
gagcgaggct ggatgagaat tgtgaccagc acctataagg gagggacagg ttccagctac 960
aacctcgcca ttgaggaggc ctgcacattt ggggacccca ttgtctaggt agatgtccct 1020
ggaagcaaca ctgtgaacca tgatgaggag gggtgattat cgacactgga tatgtccatt 1080
cagctagaaa cagtggccct catgtggaca cgaggaccag agtgtgggct gcaccccgag 1140
aggtgatagg aaaggatgag ccacaactac actggaaccc tccaccttca gacctgcttc 1200
cctccaccca cagacctgct tcctcctcca ccgtgccctg caggcctacc tccagccgcc 1260
agagaggaca gctatggtct aggacagttc tggtgttacc ctggagtcca cgggagggga 1320
actagtccag actgcctgag atgagtaaag tatctggcgt caccaaaaaa aaaaaaaaaa 1380
aaaaaaa                                                           1387

<210> 3
<211> 306
<212> PRT
<213> r
```

## Claims

**1.** Method for the development of a medicament for the treatment of stroke comprising the use of a polynucleotide sequence encoding Cathepsin Y or homologues or fragments thereof or the according protein / polypeptide.

**2.** Method according to claim 1, comprising the determination of the efficiency of a compound or agent in a model for stroke.

**3.** Method according to claim 2, whereby the efficiency of the compound /agent is determined by considering expression rate of the gene or by considering activity of the protein.

**4.** Method according to any of claims 1 to 3, comprising:

a) providing a test cell population comprising cells capable of expressing the Cathepsin Y gene or homologues or fragments thereof
b) contacting said test cell population with the test therapeutic agent;
c) detecting the expression of the Cathepsin Y gene(s) in said test cell population,
d) comparing the expression of the gene(s) in the test cell population to the expression of the gene(s) in a reference cell population whose disease stage is known; and identifying a difference in expression levels of the considered sequences, if present, in the test cell population and the reference cell population, thereby identifying a therapeutic agent for treating stroke.

**5.** Method according to any of claims 1 to 3, comprising contacting the Cathepsin Y protein with a compound /agent in presence of a polypeptide which is a target of the Cathepsin Y, determining the amino-terminal amino acid of the peptide or the amount of free amino acids in the sample after incubation, comparing the amino-terminal amino acid of the peptide or the amount of free amino acids with the result in a sample which doesn't contain the compound / agent.

**6.** Method according to claim 3 or 4, whereby the gene expression is considered or determined by PCR of cDNA, hybridisation of a sample DNA, or by detecting the according protein.

**7.** Use of a compound down regulating Cathepsin Y expression or activity for the manufacture of a medicament for the treatment of stroke.

**8.** Use according to claim 7, whereby the compound is an inhibitor of Cathepsin Y protein activity.

**9.** Use according to claim 8, whereby the compound has the general formula

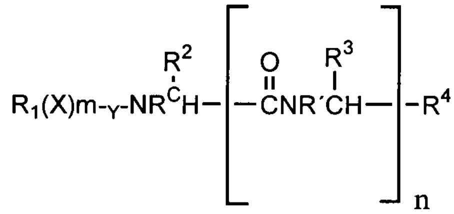

formula I

wherein:

R is selected from the group consisting of hydrogen, alkyl of from 1 to 6 carbon atoms, and where R and R2 are joined to form a ring structure of from 4 to 10 carbon atoms,
R' is selected from the group consisting hydrogen, alkyl of from 1 to 6 carbon atoms and where R' and $R^3$ are joined to form a ring structure of from 4 to 10 carbon atoms,
$R^1$ is selected from the group consisting of
alkyl of from 1 to 4 carbon atoms substituted with from 1 to 5 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur wherein said substituted alkyl group is optionally further substituted with from 1 to 2 hydroxyl groups, alkenyl of from 2 to 4 carbon atoms substituted with from 1 to 4 substituents selected from the group consisting of (a) aryl of from 6 to 10 carbon atoms, (b) aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, (c) cycloalkyl of from 3 to 8 carbon atoms and (d) heterocycles of from 3 to 14 carbon atoms having from 1 to 3 heteroatoms selected from the group consisting of nitrogen, oxygen and sulfur, aryl of from 6 to 10 carbon atoms, aryl of from 6 to 10 carbon atoms substituted with 1 to 3 substituents selected from the group consisting of alkyl of from 1 to 6 carbon atoms, aryl of from 6 to 10 carbon atoms, alkoxy of from 1 to 6 carbon atoms, aryloxy of from 6 to 10 carbon atoms, hydroxy, cyano, halo and amino, flucrenyl, heterocycles of from 3 to 14 carbon atoms having from 1 to 3 heteroatoms selected from the group consisting of nitrogen, oxygen and sulfur;
$R^2$ and $R^3$ are independently D- or L-amino acid side chains of at least 2 carbon atoms with the proviso that said amino acid side chains do not include the proline side chain;
$R^4$ is selected from the group consisting of -C(O)CH =N=N, $-CH_2OH$, -C=NOH, and $-C(O)R^5$
where $R^5$ is hydrogen, alkyl of from 1 to 6 carbon atoms, haloalkyl of from 1 to 6 carbon atoms and 1 to 2 halo groups, alkoxy of from 1 to 6 carbon atoms, $-NR^6R^7$ where $R^6$ and $R^7$ are independently selected from the group consisting of hydrogen and alkyl of from 1 to 6 carbon atoms, and aryl of from 6 to 10 carbon atoms, and $-N(CH_3)OCH_3$;
X is selected from the group consisting of -O-, $-NR^9-$, and -S- where $R^9$ is selected from the group consisting of hydrogen, alkyl of from 1 to 6 carbon atoms and aryl of from 6 to 10 carbon atoms;
Y is selected from the group consisting of -C(O)- and -C(S)-;
m is equal to zero or one; and
n is equal to zero, one or two,

or pharmaceutically acceptable salts thereof
with the proviso that when $R^1$ is 1-naphthyl, $R^2$ is $-CH(CH_3)_2$ (L-isomer), $R^3$ is $-CH_2$-Ø (L-isomer), Y is -C(O)-, m is zero and n is one, then $R^4$ is not $-N(CH_3)OCH_3$, with the further proviso that when R' is diphenylmethyl, $R^2$ is p-(benzyloxy)benzyl (L-isomer), Y is -C(O)-, and m and n are zero, then
$R^4$ is not $-N(CH_3)OCH_3$, and with still the further proviso that when $R^1$ is (1,2diphenyl)ethenyl, Y is -C(O)-, $R^2$ is $-CH_2$-Ø (L-isomer), and m and n are zero, then $R^4$ is not $-N(CH_3)OCH_3$.

**10.** Use according to claim 9, whereby the compound is selected from the group of

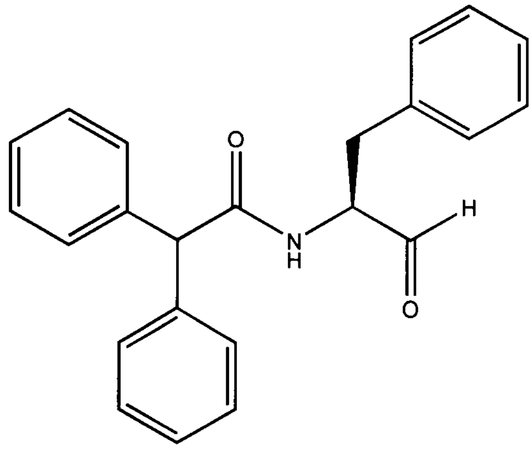

Compound 1

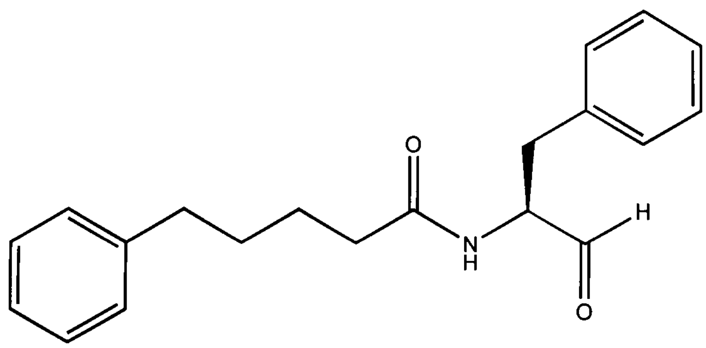

Compound 2

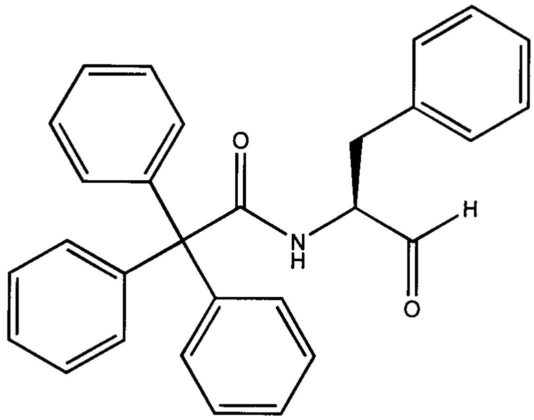

Compound 3

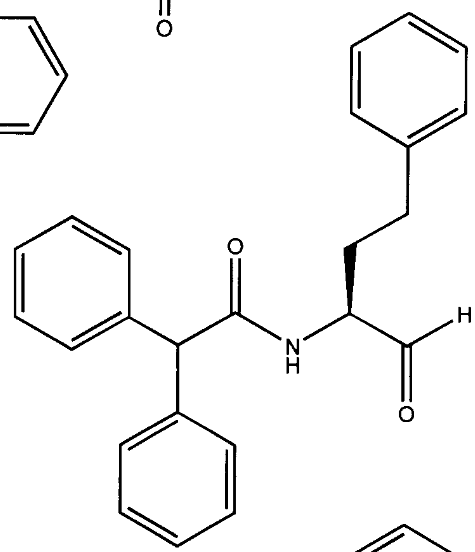

Compound 4

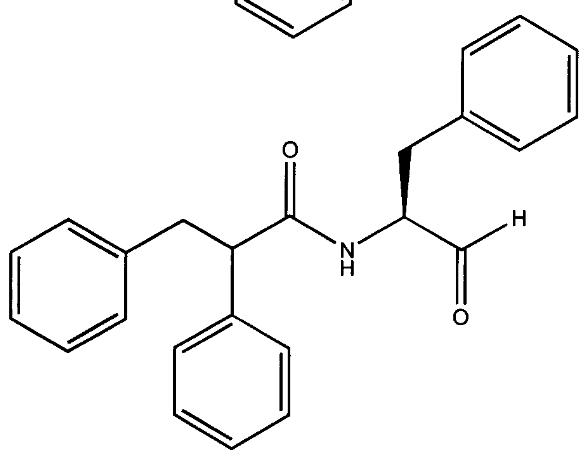

Compound 5

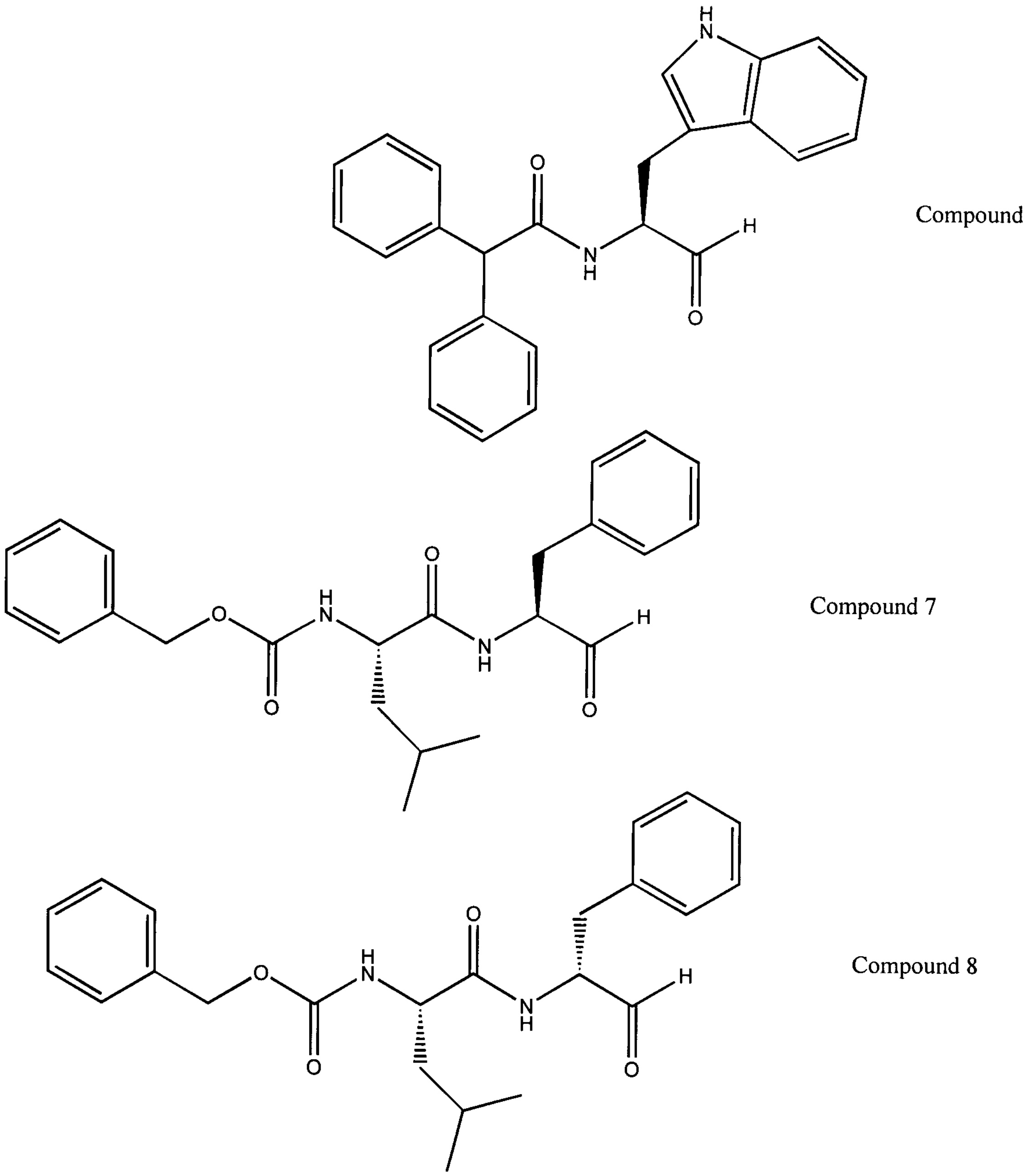

Compound 6

Compound 7

Compound 8

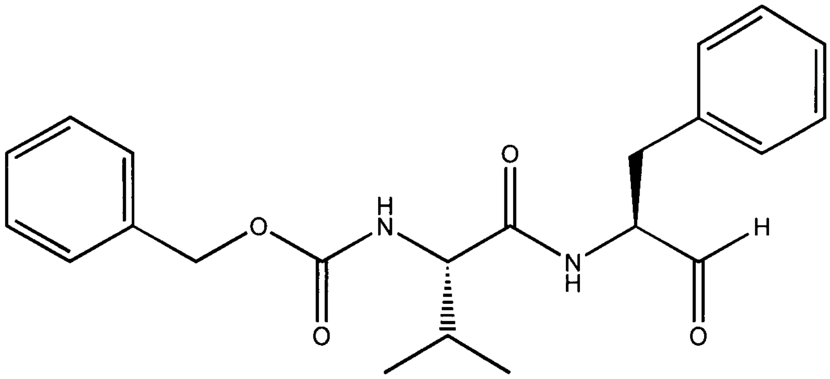

Compound 9

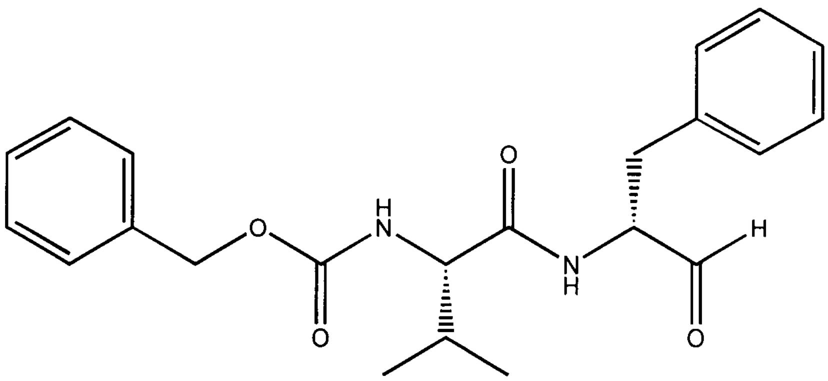

Compound 10

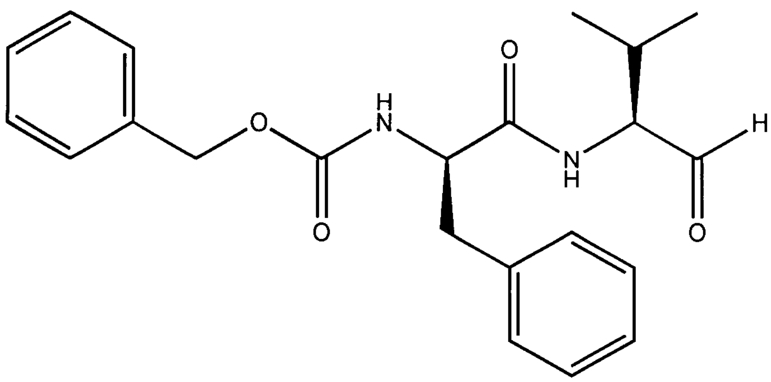

Compound 11

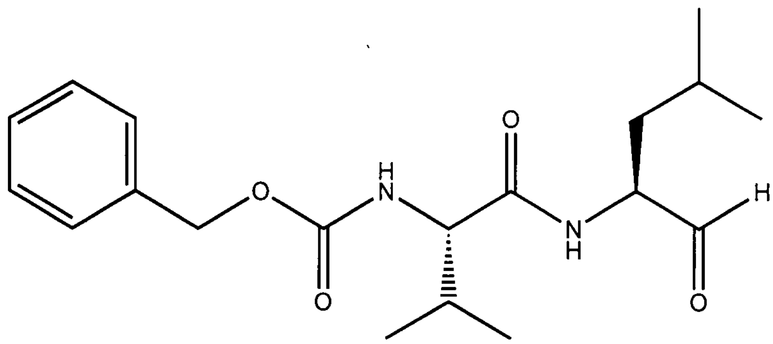

Compound 12

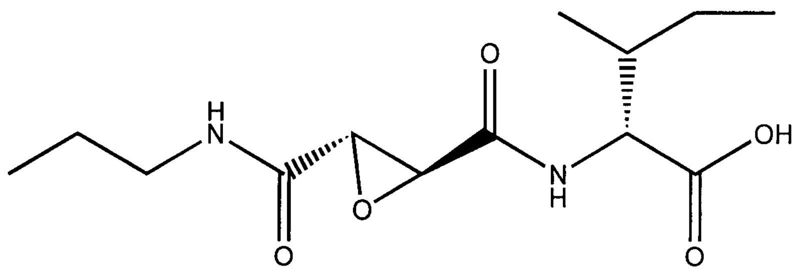

Compound 13

**11.** Method for diagnosing stroke outside of a living body or for assessing the efficacy of stroke treatment outside of a living body **characterised by** the use of a polynucleotide sequence encoding Cathepsin Y or homologues or fragments thereof or the according polypeptide.

**12.** Diagnostic kit for the method according to claim 11, whereby the kit contains one or more reagents for detecting one or more of the sequences selected from the group consisting of SEQ ID NO. 1 to 5 or homologues or fragments thereof, or antibodies directed against the protein sequences.

**13.** Use of an isolated nucleic acid sequence comprising an "antisense" sequence compared to a nucleic acid sequence encoding Cathepsin Y or a fragment of such a nucleic acid sequence as a medicament.

**14.** Method according to any of claims 1 to 6 or use according to claim 7 to 13, whereby the stroke is ischemic stroke.

**15.** Use of a transgenic animal, wherein the gene encoding Cathepsin Y is manipulated in the animal in comparison to the wild type for the investigation of stroke or of compounds influencing stroke.

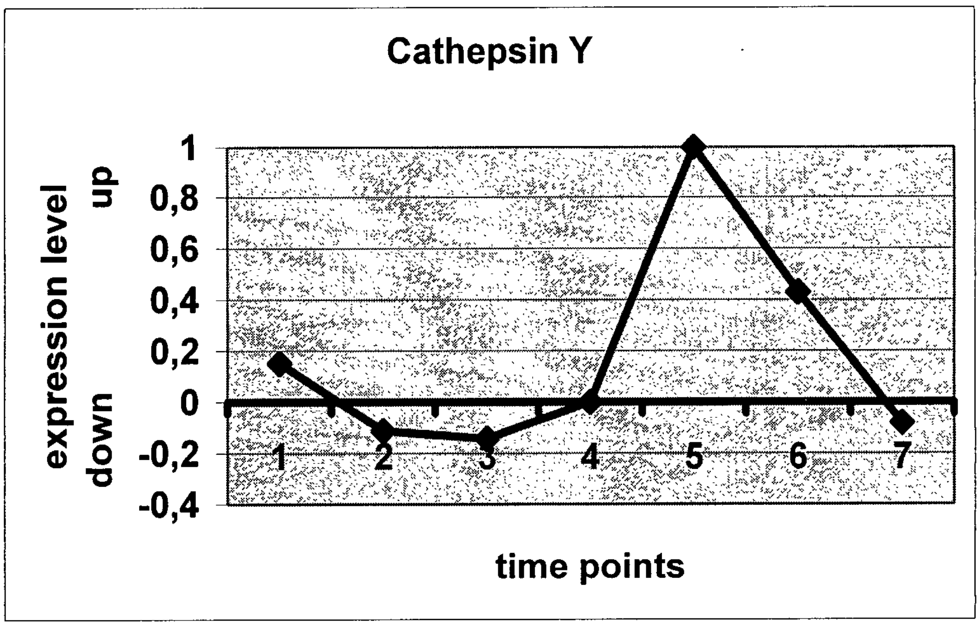
Cathepsin Y
expression level
up
down
1
0,8
0,6
0,4
0,2
0
-0,2
-0,4
1
2
3
4
5
6
7
time points

Figure 1

European Patent Office

**PARTIAL EUROPEAN SEARCH REPORT**

which under Rule 45 of the European Patent Convention shall be considered, for the purposes of subsequent proceedings, as the European search report

Application Number EP 02 00 6264

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br>Y | WO 00 27418 A (RES CORP TECHNOLOGIES INC) 18 May 2000 (2000-05-18)<br>* page 2, line 3 - page 3, line 16 *<br>* page 48, line 7-13 *<br>* page 55, line 24 - page 56; claims 1,27,28,30 * | 1-4,6, 11-15<br>1-15 | C12Q1/37<br>G01N33/68<br>A61K31/00<br>A61K38/00 |
| D,X<br><br>Y | WO 96 39194 A (ATHENA NEUROSCIENCES INC) 12 December 1996 (1996-12-12)<br>* page 2, line 9-11; claims 1,7-20,23,30 * | 12,13,15<br><br>1-15 | |
| A | GREEN 1 C D ET AL: "Open systems: panoramic views of gene expression"<br>JOURNAL OF IMMUNOLOGICAL METHODS, ELSEVIER SCIENCE PUBLISHERS B.V.,AMSTERDAM, NL,<br>vol. 250, no. 1-2,<br>1 April 2001 (2001-04-01), pages 67-79, XP004230695<br>ISSN: 0022-1759<br>* page 76, column 2 - page 77 * | 1 | |
| | -/-- | | |

TECHNICAL FIELDS SEARCHED (Int.Cl.7)

C12Q
G01N
A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 16 September 2002 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

## INCOMPLETE SEARCH SHEET C

Application Number
EP 02 00 6264

Claim(s) searched incompletely:
7-9

Reason for the limitation of the search:

Present claims 7-9 relate to the use of an extremely large number of compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds mentioned in mentioned in claim 10.

European Patent Office

# PARTIAL EUROPEAN SEARCH REPORT

Application Number
EP 02 00 6264

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| D,A | THERRIEN CHRISTIAN ET AL: "Cathepsins X and B can be differentiated through their respective mono- and dipeptidyl carboxypeptidase activities." BIOCHEMISTRY, vol. 40, no. 9, 6 March 2001 (2001-03-06), pages 2702-2711, XP002212082 ISSN: 0006-2960 * figures 1,6 * --- | 10 | |
| A | KOZIKOWSKI A P ET AL: "Design of remarkably simple, yet potent urea-based inhibitors of Glutamate carboxypeptidase II (NAALADase)" JOURNAL OF MEDICINAL AND PHARMACEUTICAL CHEMISTRY, AMERICAN CHEMICAL SOCIETY. EASTON, US, vol. 44, no. 3, 1 February 2001 (2001-02-01), pages 298-301, XP002196918 * page 298, column 1 * * page 300, column 1 * ----- | 1 | TECHNICAL FIELDS SEARCHED (Int.Cl.7) |

EPO FORM 1503 03.82 (P04C10)

**ANNEX TO THE EUROPEAN SEARCH REPORT ON EUROPEAN PATENT APPLICATION NO.** EP 02 00 6264

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2002

| Patent document cited in search report | | Publication date | Patent family member(s) | | Publication date |
|---|---|---|---|---|---|
| WO 0027418 | A | 18-05-2000 | EP | 1131082 A2 | 12-09-2001 |
| | | | WO | 0027418 A2 | 18-05-2000 |
| WO 9639194 | A | 12-12-1996 | US | 5783434 A | 21-07-1998 |
| | | | US | 5849711 A | 15-12-1998 |
| | | | CA | 2221684 A1 | 12-12-1996 |
| | | | EP | 0831920 A1 | 01-04-1998 |
| | | | JP | 11506923 T | 22-06-1999 |
| | | | WO | 9639194 A1 | 12-12-1996 |
| | | | US | 5858982 A | 12-01-1999 |

EPO FORM P0459

For more details about this annex : see Official Journal of the European Patent Office, No. 12/82